Europäisches Patentamt

European Patent Office

Office européen des brevets

(19)

(11) Publication number: **0 166 522 B1**

# (12) EUROPEAN PATENT SPECIFICATION

(45) Date of publication of patent specification: **24.07.91**

(51) Int. Cl.5: **A61K 7/047**, A61K 7/50, A61K 7/48

(21) Application number: **85303536.8**

(22) Date of filing: **20.05.85**

(54) Cosmetic preparation.

(30) Priority: **24.05.84 ZA 843939**

(43) Date of publication of application:
**02.01.86 Bulletin 86/01**

(45) Publication of the grant of the patent:
**24.07.91 Bulletin 91/30**

(84) Designated Contracting States:
**AT BE CH DE FR GB IT LI NL SE**

(56) References cited:
**DE-A- 1 288 749**
**DE-A- 1 492 194**
**FR-A- 2 192 798**
**US-A- 4 052 513**

**"HANDBUCH DER KOSMETIKA UND RIECH-
STOFFE", vol. 3, H. JANISTYN: "Die Körperp-
flegemittel", 2nd edition, 1973, pages
935-937.**

**CHEMICAL ABSTRACTS, vol. 90, no. 8, Feb-
ruary 1979, page 379, abstract no. 61078m,
Columbus, Ohio, US & PL-A-84 887
(ZJEDNOCZONE ZESPOLY GOSPODARCZE,
WARSZAWA) 30-11-1976**

PATENTS ABSTRACTS OF JAPAN, vol. 8, no.
222 (C-246)[1659], 9th October 1984; & JP - A
- 59 106 411 (KANEBO K.K.) 20-06-1984

(73) Proprietor: **Pegel, Karl Heinrich**
**Dept. of Chemistry University of Natal King
George V Avenue
Durban Natal(ZA)**

(72) Inventor: **Papaphilippou, Alexander Philip
106 Ocean Drive
Athlone Park Amanzimtoti Natal(ZA)**

(74) Representative: **Perry, Robert Edward et al
GILL JENNINGS & EVERY 53-64 Chancery
Lane
London WC2A 1HN(GB)**

EP 0 166 522 B1

**Description**

This invention relates to a cosmetic cleansing cream composition and to its use for removing nail varnish and cleansing skin.

Conventional nail polish removers, such as acetone, cause dehydration and dewaxing. They remove natural lipids from the nails and surrounding cuticles. They also evaporate and spill easily. These disadvantages have been alleviated to an extent by providing a gel containing acetone with other additives such as colourants, vitamins and proteins. However, the main disadvantage persists. It is an object of the present invention to provide a composition which not only removes nail varnish effectively, but which also acts as a moisturiser and tissue care agent.

US-A-4052513 discloses a topical anaesthetic composition comprising diesters of dicarboxylic acids.

FR-A-2192798 discloses an anti-perspirant composition comprising a diester of a dicarboxylic acid and a propellant.

Handbuch der Kosmetika und Riechstoffe, vol. 3, second edition (1973) 935-937, discloses a nail varnish remover comprising a diester. Analogous compositions are described in C.A. 90 (8) (February 1979) 61078m and US-A-2765257.

A cosmetic cleansing cream composition according to the invention comprises from 40 to 60% diethyl succinate, from 5 to 10% lipids, from 9.3 to 39% water, from 0 to 13.9% moisturisers, and from 7 to 21.3% emulsifiers, the percentages being by weight. The composition can be used to remove nail varnish, and improve cuticle texture, or to cleanse skin, and also to act as a conditioner for beauty care and industrial purposes.

The diester, i.e. diethyl succinate, used in the novel composition may be present in a solid or semi-solid matrix as an oil-in-water or water-in-oil emulsion. The solid or semi-solid matrix, besides being a good vehicle for the diester, provides the lipid or fatty "nourishing" material which counteracts the lipid-stripping effect of the diester, thereby avoiding any tissue hardening and damage which provides access to attack by micro-organisms.

Diethyl succinate is not water-soluble, and therefore cannot act as a dehydrating agent. On the contrary, the water content of the composition provides a moisturising effect.

The compositions of the invention contain one or more lipids such as lanolin, various vegetable and animal oils and fats, or sterols. They may also contain moisturisers such as glycerol or monoalkyl ethers of ethylene and propylene glycols.

In addition the matrix may contain surfactants (non-ionic, anionic, cationic) to provide not only emulsifying properties, but also water solubility of the emulsion on removal.

The preferred lipids of the invention are lanolin, plant oils and fats, paraffin (hard, soft, or liquid), cholesterol and sitosterol.

The preferred emulsifiers of the invention are emulsifying wax (B.P.), or (U.S.P), glycerin monostearate (self emulsifying), Span 60 (10-80), Tween 60 (10-80), polyoxyethylene sterols as primary emulsifiers, and sodium lauryl sulphates as secondary emulsifiers or equivalent emulsifiers according to the state of the art.

The preferred moisturisers are monomethyl ethers of ethylene - and propylene glycol. Equivalent moisturisers according to the state of art may be substituted.

Any acceptable preservative can be used, such as butylated hydroxy = anisole (BHA and butylated hydroxytoluene (BHT).

The nail varnish cleansing and tissue conditioning cosmetic may be applied to the nail surface and surrounding tissue with gentle massage for a period of up to two minutes before the treated area is either wiped or rinsed with water; the process can be repeated.

Use of the compositions of the invention not only prevents hardening, embrittlement and cracking of the nails, cuticles and surrounding skin, but ensures that these tissues remain supple and well nourished and remain free from microbial attack and physical damage due to tissue degradation.

The cosmetic products of this invention also act as cleansing agents for the removal of foreign matter such as make-up, (with the possible exception of eye make-up), industrial grime of varied origin, paint, and dirt in general. By replenishing essential moisture and lipid nutrients to skin tissue, these products prevent skin hardening and thus premature aging.

The products of the invention not only achieve the "cleansing" process in an effective and agreeable manner, but at the same time prevent aggressive stripping of moisture and lipids and provide concomitant nail and skin care. Indeed experience has shown that there is a noticeable prevention in nail embrittlement and improvement in texture of the nails and surrounding tissue. An added bonus is the safety aspect since the viscous nature of the products, which ranges from a thick oil to a soft creamy consistency, are spill-resistant, and the "solvents" do not evaporate rapidly.

2

EXAMPLE 1

| A | Diethyl succinate | 46.3% |
|---|---|---|
| | Lanoline (anhydrous) | 4.6% |
| | Hard paraffin | 4.6% |
| | Emulsifying wax (B.P.) | 4.6% |
| | Glycerin monostearate (self-emulsifying) | 16.7% |
| B | Propylene glycol monomethyl ether | 13.9% |
| | Water | 9.3% |

Heat A and B separately to 70-75° C.
Add B to A slowly, and cool gradually with agitation; add preservative, colourant and perfume, as required, at 50° C.

NOTES

a) Anhydrous lanolin may be substituted by water soluble or liquid lanolin, hydrogenated wool fat, various sterols, and the lip up to 20% but preferably between 3 and 10%.
b) Hard paraffin may be substituted by liquid or soft paraffin, or vegetable or animal oil and fats ranging between 1 and 20% but preferably between 2 and 10%.
c) The emulgent may consist of an appropriate combination of non-ionic, anionic or cationic surfactants to achieve the desired hydrophilic to lipophilic balance of 4 to 12, preferably between 6 and 8.
d) The propylene glycol monomethyl ether may be replaced by glycerol and glycols and their derivatives with humectant properties in the range of 5 to 15%.
e) The water content is adjusted accordingly to 100%. Preservative addition is optional but, if more than 10% water is included, a preservative may be added up to 0.5%.

EXAMPLE 2

| | |
|---|---|
| Diethyl succinate | 40-45% |
| Lanolin (anhydrous) | 10-13% |
| Hard paraffin | 4-5% |
| Span 60 | 6-7% |
| Tween 60 | 2-3% |
| Sodium lauryl sulphate | 1-2% |
| Preservative | 0.1% |
| Water | to 100% |

The compositions of further Examples (3 to 12) of the invention are tabulated below. Amounts are given in percentages by weight.

| Example | 3 | 4 | 5 | 6 | 7 | 8 | 9 | 10 | 11 | 12 |
|---|---|---|---|---|---|---|---|---|---|---|
| Diethyl succinate | 50 | 50 | 50 | 50 | 50 | 60 | 50 | 50 | 50 | 60 |
| Anhydrous lanolin | 5 | 3 | 5 | 4 | 4 | 1 | – | – | 5 | 5 |
| Hard paraffin | 5 | 3 | – | 4 | 4 | 2 | 4 | 3 | 4 | 3 |
| Span 60 | – | 10 | – | – | 8 | – | 8 | – | 11 | – |
| Emulsifying wax (B.P.) | – | – | – | – | – | – | – | – | 5 | – |
| Beeswax | – | – | – | – | – | – | – | 4 | – | – |
| Stearic acid | – | – | – | – | – | 2 | – | – | – | – |
| Wood alcohols | – | – | – | – | – | – | 4 | – | – | – |
| Glycerin monostearate (self-emulsifying B.P.) | 10 | – | 5 | 8 | – | 5 | – | 5 | – | 8 |
| Ethylene glycol monomethyl ether | 10 | 10 | 10 | – | – | – | – | 10 | 10 | – |
| Generol E-5 | 5 | 5 | 5 | 4 | 4 | 2 | 4 | 3 | – | 4 |
| Water | 15 | 19 | 25 | 30 | 30 | 28 | 30 | 25 | 15 | 20 |

## Claims

1. A cosmetic cleansing cream composition, suitable for nail varnish removal and skin cleansing, which comprises from 40 to 60% diethyl succinate, from 5 to 10% lipids, from 9.3 to 39% water, from 0 to 13.9% moisturisers, and from 7 to 21.3% emulsifiers, the percentages being by weight.

2. A nail varnish removing method, which comprises applying to the nail a composition according to claim 1.

3. A skin cleansing method, which comprises applying to the skin a composition according to claim 1.

## Revendications

1. Une crème démaquillante, approprié à enlever le vernis d'ongles et pour démaquiller la peau, qui compris de 40 à 60% succinat diethyle, de 5 à 10% lipides, de 9.3 à 39% de l'eau, de zero à 13.9% de la crème hydratante, et de 7 à 21.3% emulsifiers, les pourcentages étant au poids.

2. Une méthode pour enlever le vernis d'ongles, qui compris appliquer à l'ongle une crème selon revendication 1.

3. Une méthode pour démaquiller la peau, qui compris appliquer à la peau une crème selon revendication 1.

## Patentansprüche

1. Eine Zubereitung einer kosmetischen Creme, nützlich für die Entfernung von Nagellack und zur Reinigung der Haut, die von 40 bis 60% Diäthylsuccinat, von 5 bis 10% Lipide, von 9,3 bis 39% Wasser, von 0 bis 13,9% feuchtigkeits Vermittler, und von 7 bis 21,3% Emulgatoren im prozentualem Gewichtsverhältnis enthält.

2. Ein Nagellack entfernungs Vorgang in dem auf dem Nagel eine Zubereitung folgens Anspruch 1 angewendet wird.

3. Ein Haut säuberungs Vorgang in dem auf der Haut eine Zubereitung folgens Anspruch 1 angewendet wird.